# EUROPÄISCHE PATENTSCHRIFT

(11) **EP 0 775 105 B1**
(45) Veröffentlichungstag und Bekanntmachung des Hinweises auf die Patenterteilung: **03.03.1999**
(21) Anmeldenummer: 95930429.6
(22) Anmeldetag: 29.07.1995
(51) Int. Cl.: C07C 231/24

(54) **VERFAHREN ZUR HERSTELLUNG VON POLYHYDROXYFETTSÄUREAMID-LÖSUNGEN MIT GUTER FARBQUALITÄT UND DEREN VERWENDUNG**
METHOD FOR PREPARING POLYHYDROXY FATTY ACID AMIDE SOLUTIONS OF GOOD COLOR QUALITY AND THEIR USE
PROCEDE DE PREPARATION DE SOLUTIONS D'AMIDE D'ACIDE GRAS DE POLYHYDROXY DE BONNE QUALITE DE COULEUR ET LEUR UTILISATION

(30) Priorität: 02.08.1994 DE 4427301
(43) Veröffentlichungstag der Anmeldung: 28.05.1997
(73) Patentinhaber: Clariant GmbH, 65929 Frankfurt am Main (DE)
(72) Erfinder: PAPENFUHS, Bernd, D-84524 Neuötting (DE); VYBIRAL, Reinhard, D-84508 Burgkirchen (DE)
(86) Internationale Anmeldenummer: EP9503020
(87) Internationale Veröffentlichungsnummer: WO9604236

(56) Entgegenhaltungen:
- WO-A-93/09215
- GB-A- 919 428

## Beschreibung

Die Erfindung betrifft ein Verfahren zur Herstellung von farbverbesserten Lösungen von Polyhydroxyfettsäureamid, das durch Umsetzung von N-Alkylpolyhydroxyamin und Fettsäurealkylester erhalten worden ist. Die Erfindung betrifft ferner die Verwendung dieser Lösungen.

Polyhydroxyfettsäureamide sind wertvolle und vielfach einsetzbare oberflächenaktive Verbindungen. So können sie zum Beispiel als solche oder in Mischung mit anionischen, kationischen und/oder nichtionischen Tensiden als Reinigungsmittel, Waschmittel, Textilbehandlungsmittel und dergleichen eingesetzt werden, und zwar in Form von Festprodukten (zum Beispiel als Pulver, Körner oder Granulat), Lösungen, Dispersionen, Emulsionen, Pasten und dergleichen. Da Polyhydroxyfettsaureamide auch gut biologisch abbaubar sind und aus nachwachsenden Rohstoffen hergestellt werden können, haben sie in jüngster Zeit größere Bedeutung erlangt.

Bei den in Rede stehenden Polyhydroxyfettsäureamiden handelt es sich in der Regel um Verbindungen der allgemeinen Formel R-CO-NR'-Z, in der R einen Kohlenwasserstoffrest mit etwa 5 bis 30 C-Atomen, vorzugsweise 8 bis 18 C-Atomen, R' H, Alkyl oder Hydroxyalkyl mit bis zu vorzugsweise 8 C-Atomen und Z einen Polyhydroxykohlenwasserstoffrest mit mindestens drei OH, die auch alkoxyliert sein können, vorzugsweise einen Zuckeralkoholrest, bedeuten. Die bevorzugten Polyhydroxyfettsäureamide entsprechen also der nachstehenden Formel 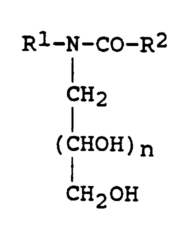 worin R¹ ein kurzkettiges Alkyl wie C₁ bis C₄-Alkyl oder Hydroxyalkyl wie -CH₂CH₂OH, R² ein Fettalkyl und n 3 oder 4 ist. Die Verbindungen mit n = 4, die besonders bevorzugt sind, werden als Glycamide bezeichnet und im Falle von Glucose als Hexose-Rest Glucamide. Die Fettsäurealkylester sind im allgemeinen Fettsäure-C₁ bis C₄-alkylester, wobei Methyl, Ethyl, Propyl oder Isopropyl bevorzugt sind. Die Fettsäuremethylester sind besonders bevorzugt. Der Fettsäurerest (die Acylgruppe) hat im allgemeinen 6 bis 24 C-Atome, vorzugsweise 8 bis 18 C-Atome. Er kann gesättigt oder ungesättigt (vorzugsweise ein- bis dreifach ungesättigt) sein.

Die Herstellung von Polyhydroxyfettsäureamiden erfolgt im allgemeinen durch Umsetzung von einem N-Alkylpolyhydroxyamin (zum Beispiel N-Alkylglucamin) mit einem Fettsäurealkylester in Gegenwart basischer Katalysatoren, wobei die Umsetzung in Schmelze (in Substanz) oder mit Hilfe von Lösungsmitteln durchgeführt wird. Die nachstehende Reaktionsgleichung mit N-Methylglucamin und Laurinsäuremethylester soll dies näher veranschaulichen:

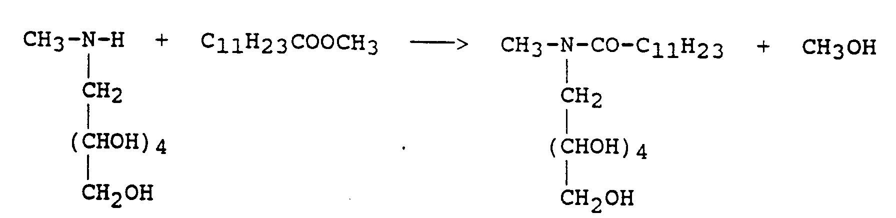

Die Durchführung der Umsetzung in Gegenwart von Lösungsmitteln ist im allgemeinen bevorzugt.

Derartige Herstellungsverfahren sind in einer Reihe von Druckschriften beschrieben, wovon hier stellvertretend WO 92/06073 und WO 94/10130 genannt seien. Das N-Alkylpolyhydroxyamin und der Fettsäurealkylester werden in einer im wesentlichen äquimolaren Menge eingesetzt. Die Umsetzung wird bei einer Temperatur von bis zu etwa 135 °C durchgeführt. Als basische Katalysatoren werden vorzugsweise Alkalimetallhydroxide, Alkalimetallcarbonate und/oder C₁ bis C₄-Alkalimetallalkoxide wie Natriummethylat und Kaliummethylat eingesetzt und als Lösungsmittel C₁ bis C₄-Alkohole, Glycerin und/oder Glykole wie Ethylenglykol und Propylenglykol (1,2-Propylenglykol und 1,3-Propylenglykol). Die Katalysatormenge liegt bei 0,1 bis 20 Mol-%, bezogen auf Fettsäureester. Das Lösungsmittel wird in einer solchen Menge eingesetzt, daß die erhaltene Lösung von Polyhydroxyfettsäureamid 10 bis 80gew.%ig ist. Wird das Lösungsmittel zweckmäßigerweise unter Verwendung von Vakuum entfernt, erhält man das Polyhydroxyfettsäureamid in fester Form, zum Beispiel als Pulver. Die erhaltenen Polyhydroxyfettsäureamid-Produkte, ob in fester Form oder in Form von Lösungen, weisen nicht die gewünschte Farbqualität auf, sie sind vielmehr im allgemeinen hell- bis dunkelbraun gefärbt.

Als Stand der Technik sei auch noch die Druckschrift WO 93/09215 erwähnt, in der flüssige farbstabilisierte Detergenzformulierungen beschrieben werden, unter anderem auch solche mit Polyhydroxyfettsäureamiden. Der Schutz gegen Farbveränderung der frischen Formulierungen wird dadurch erreicht, daß den Formulierungen 0,001 bis 10 Gew.-%, bezogen auf die gesamte Formulierung, von einer oder mehreren der folgenden farbstabilisierenden Verbindungen zugesetzt werden: Sulfite, Hydrogensulfite oder Pyrosulfite, Schwefeldioxid, schwefelige Säure, α-Hydroxyalkylsulfonsäuren, Mercaptoethanol, Natriummercaptoacetat, 2-Aminoethanthiol, Cystein, Polycystein, Glutathion und Formamidinsulfinsäure.

Die Aufgabe der vorliegenden Erfindung besteht nun darin, die nach den bekannten Verfahren aus N-Alkylpolyhydroxyaminen und Fettsäureestern im allgemeinen in Form von Lösungen hergestellten mehr oder weniger braungefärbten Polyhydroxyfettsäureamide aufzuhellen, das heißt in praktisch farblose Produkte überzuführen.

Es wurde überraschenderweise gefunden, daß man Polyhydroxyfettsäureamide mit guter Farbqualität erhält, wenn man in das zu entfärbende Produkt (Rohprodukt) dithionige Säure und/oder Derivate davon inkorporiert. Dies ist ein unerwartetes Ergebnis, zumal - wie die später angeführten Vergleichsbeispiele zeigen - mit anderen ebenfalls reduzierend wirkenden Schwefelverbindungen, zum Beispiel mit jenen, die in der obengenannten WO 93/09215 empfohlen werden, der angestrebte Effekt nicht erreicht wird. Es war in der Tat nicht vorhersehbar, daß gerade und nur mit Schwefelverbindungen aus der Gruppe der dithionigen Säure Polyhydroxyfettsäureamid in einem hohen Ausmaß farbverbessert werden kann.

Das erfindungsgemäße Verfahren zur Herstellung einer farbverbesserten Lösung von Polyhydroxyfettsäureamid aus N-Alkypolyhydroxyamin und Fettsäurealkylester ist demnach dadurch gekennzeichnet, daß man in die Rohlösung mindestens eine Schwefelverbindung aus der Gruppe der dithionigen Säure in einer wirksamen Menge einmischt.

Bevorzugte Verbindungen aus der Gruppe der dithionigen Säure sind die dithionige Säure selbst (H₂S₂O₄) und deren Salze, die primärer Art (saure Salze) oder sekundärer Art sein können. Die Salze umfassen zum Beispiel Ammoniumsalze, Alkanolammoniumsalze und Metallsalze, vorzugsweise von Alkali- und Erdalkalimetallen. Besonders bevorzugte Verbindungen sind die Ammoniumdithionite und Alkalimetalldithionite wie Kalium- und Natriumdithionit. Die Schwefelverbindungen können als solche oder in Form einer Lösung eingesetzt werden, vorzugsweise als wäßrige oder wäßrig/alkoholische (C₁ bis C₃-Alkanol) Lösung 10 bis 50gew.%ig.

Die Menge an erfindungsgemäß einzusetzender Schwefelverbindung kann in weiten Grenzen variieren. Aufgrund der unerwartet hohen Wirksamkeit wird häufig auch schon mit sehr wenig Dithionitverbindung der angestrebte bleichende Effekt erreicht. Die wirksame Menge an erfindungsgemäß einzusetzenden Verbindungen liegt demnach im allgemeinen bei 0,005 bis 1 Gew.-%, vorzugsweise bei 0,05 bis 0,15 Gew.-%, Gewichtsprozente bezogen auf die zu bleichende Lösung.

Die erfindungsgemäß zu behandelnden Lösungen sind - wie bereits erwähnt - in der Regel solche, die bei der Herstellung von Polyhydroxyfettsäureamiden durch Umsetzung von Alkylpolyhydroxyaminen mit Fettsäureestern anfallen und mehr oder weniger braungefärbt sind. Erfindungsgemäß kann man auch solche Lösungen behandeln, die durch Auflösen von unreinem (gefärbtem) Polyhydroxyfettsäureamid erhalten werden. Das Lösungsmittel besteht vorzugsweise aus Wasser, einem niedrigen Alkohol wie C₁ bis C₄-Alkanol, einem niedrigen Glykol wie Ethylenglykol und Propylenglykol oder Glycerin oder aus einer Mischung davon, zum Beispiel einer Mischung aus Wasser und Alkohol, Wasser und Glykol, Alkohol und Glykol oder Wasser, Alkohol und Glykol. Die zu reinigenden Lösungen enthalten im allgemeinen 10 bis 80 Gew.-% Polyhydroxyfettsäureamid, vorzugsweise 20 bis 60 Gew.-%, bezogen auf das Gewicht der Lösung. Die Lösungen sind in der Regel neutral oder alkalisch. Ihr pH-Wert liegt also im allgemeinen im Bereich von etwa 6 bis 12, vorzugsweise von etwa 7 bis 11.

Die zu entfärbenden Lösungen werden erfindungsgemäß mit den beschriebenen Schwefelverbindungen versetzt, zweckmäßigerweise unter Rühren und unter Aufrechterhalten der genannten pH-Werte, wobei ein pH-Wert von 7 bis 11 bevorzugt ist. Das Einmischen kann man bei Raumtemperatur oder einer erhöhten Temperatur vornehmen, das heißt im Bereich von etwa 20 bis 100 °C, vorzugsweise 40 bis 80 °C, und bei Atmosphärendruck oder dem je nach Lösungsmittel und Temperatur sich einstellenden Druck. Die Schwefelverbindungen können auf einmal, portionsweise oder kontinuierlich in die Lösung eingebracht werden. Nach dem Einmischen wird man - sofern die gewünschte Entfärbung noch nicht erreicht ist - bei der angegebenen Temperatur und unter Rühren nachreagieren lassen, bis die gewünschte Farbqualität und damit die angestrebte farblose Lösung vorliegt. Falls farbloses Polyhydroxyfettsäureamid als solches gewünscht wird, kann man es aus den erfindungsgemäß erhaltenen Lösungen, zum Beispiel durch Abdampfen oder Abdestillieren des Löungsmittels in Kombination mit Filtrieren oder Abschleudern isolieren und gewinnen. Abdampfen oder Abdestillieren wird vorzugsweise bei einer Temperatur von 50 bis 100 °C unter Anwendung eines Vakuums durchgeführt.

Die mit dem erfindungsgemäßen Entfärbungsverfahren erhaltenen Polyhydroxyfettsäureamid-Lösungen und das daraus gegebenenfalls gewonnene Polyhydroxyfettsäureamid (Pulver, Paste und dergleichen) sind praktisch farblos (wasserhell). Die gute Farbqualität bleibt auch bei Lagerung des Produktes erhalten. Die Produkte sind ferner geruchsneutral, da aufgrund der hohen Wirksamkeit der empfohlenen Dithionit-Verbindungen im allgemeinen eine nur sehr kleine Menge zur Erreichung der angestrebten Bleichung benötigt wird. Das erfindungsgemäße Verfahren führt also zu Polydydroxyfettsäureamid-Produkten mit guter Farbqualität, Geruchsneutralität und einer langandauernden Stabilität gegen Verfärbung oder Farbänderung.

Die Erfindung wird nun an Beispielen und Vergleichsbeispielen noch näher erläutert. Die benutzten Abkürzungen NMG und GA stehen für N-Methylglucamin beziehungsweise N-Methylglucamid.

### Beispiel 1

1. Herstellung einer GA-Lösung nach Verfahren des Standes der Technik:
   In einem mit Rührer, Thermometer und Rückflußkühler ausgestatteten Reaktionsgefäß werden 97,6 g NMG (0,5 mol) und 22 g Propylenglykol vorgelegt und auf 125 °C erhitzt. Bei dieser Temperatur werden 115,5 g (0,53 mol) C₁₂ bis C₁₄-Fettsäuremethylester und anschließend 7,0 g Natriummethylat (das sind 8 Mol-% bezogen auf NMG) dazugegeben, worauf die Mischung bei einer Temperatur von etwa 100 °C gehalten wird. Das durch die Reaktion entstehende Methanol wird Zunächst im System belassen. Nach Erreichen eines Umsetzungsgrades von etwa 70 Gew.-% GA wird ein Vakuum von 60 mbar angelegt und bei einer Temperatur von 85 bis 88 °C unter Abdestillieren des Methanols weiterreagiert, bis nach etwa 70 Minuten 92 Gew.-% GA gebildet sind, bezogen auf den Feststoffanteil der Lösung. Nach Aufheben des Vakuums wird das Reaktionsgemisch in 71,0 g Wasser und 33,0 g Ethanol aufgenommen. Die erhaltene GA-Lösung mit Wasser, Propylenglykol und Ethanol als Lösungsmittel ist dunkelbraun gefärbt. Sie hat einen pH-Wert von 10 und die Konzentration an GA beträgt 55 Gew.-%. Von dieser Lösung werden zwei Portionen zu je etwa 130 g gebildet, und eine der beiden Portionen wird mit Citronensäure auf einen pH-Wert von 8,3 eingestellt. Es liegen also zwei dunkelbraun gefärbte GA-Lösungen mit einem pH-Wert von 10 [Rohlösung a)] und 8,3 [Rohlösung b)] vor.
2. Erfindungsgemäße Behandlung der braunen GA-Lösungen a) und b):
   Beide Rohlösungen mit jeweils 130 g und mit einer GA-Konzentration von 55 Gew.-% werden auf 60 °C erhitzt und bei dieser Temperatur mit jeweils 0,13 g festem Natriumdithionit unter Rühren versetzt, das sind 0,1 Gew.-% Natriumdithionit, bezogen auf das Gewicht der Lösung, worauf eine halbe Stunde bei einer Temperatur von etwa 60 °C nachgerührt und anschließend abgekühlt wird. Die Lösung a) wird nun ebenfalls mit Citronensäure auf einen pH-Wert von 8,3 gebracht.
   Die nun vorliegenden Lösungen a) und b) sind - wie schon mit dem freien Auge festgestellt werden kann - nahezu farblos. Sie weisen ferner keinen Geruch bezüglich Schwefelverbindungen auf. Zur zahlenmäßigen Darstellung der hervorragenden Farbqualität der beiden erfindungsgemäß gebleichten Lösungen werden ihre Lichttransmissionswerte ermittelt und mit dem in gleicher Weise bestimmten Transmissionswert der braunen Ausgangslösung verglichen. Zur Bestimmung der Transmissionen wird unter Verwendung von 50vol.%igem wäßrigem Methanol eine 25 Gew.-% GA enthaltende Lösung hergestellt und bei 420 nm in einer 1-cm-Küvette vermessen. Die dabei erhaltenen Werte werden anschließend auf eine 50gew.%ige GA-Lösung umgerechnet. Die Ergebnisse sind nachstehend zusammengefaßt:
   Lichttransmissionswerte:
   ungebleichte Lösung: 65,1 %
   nach der erfindungsgemäßen Behandlung:
   Lösung a): 79,8 %
   Lösung b): 77,6 %

### Beispiel 2

1. Herstellung von C₁₂-N-Methylglucamid nach Verfahren des Standes der Technik:
   In einem Reaktionsgefäß werden 219 g (1,0 mol) C₁₂-Fettsäuremethylester vorgelegt und auf 100 °C erhitzt. Nun werden parallel 195 g (1,0 mol) einer NMG-Schmelze (130 °C) und 18 g einer 30gew.%igen methanolischen Natriummethylat-Lösung (0,1 mol enthaltend) bei circa 0,1 bar und 100 °C innerhalb 1 Stunde zugegeben. Das in der Reaktionsmischung enthaltene und ständig neu gebildete Methanol wird im Vakuum kontinuierlich entfernt, wobei die Viskosität der Mischung allmählich ansteigt. Nach vollständiger Abdestillation des Lösungsmittels wird mit 9,4 g Citronensäure versetzt und so schließlich ein braunes wachsartiges Produkt erhalten.
2. Erfindungsgemäße Behandlung des so erhaltenen Glucamids:
   Vom erhaltenen Produkt wird bei 60 °C eine 55 Gew.-% GA, 20 Gew.-% Propylenglykol und 25 Gew.-% Wasser enthaltende Lösung hergestellt und Teile dieser Lösung bei derselben Temperatur mit 0,05 beziehungsweise 0,2 Gew.-%, bezogen auf die Lösung, Natriumdithionit 1 Stunde lang behandelt. Die abgekühlten Mischungen [(1): ungebleicht;
   (2): 0,05 Gew.-% Dithionit; (3): 0,2 Gew.-% Dithionit] weisen folgende Lichttransmissionswerte (analog Beispiel 1 bestimmt) auf:
   (1): 50,4 %
   (2): 71,4 %
   (3): 78,7 %

   Durch zweiwöchige Lagerung bei 40 °C kann die Transmission sogar noch deutlich erhöht werden, wohingegen die unbehandelte Probe dunkler wird:
   (1): 48,5 %
   (2): 75,9 %
   (3): 89,5 %

### Vergleichsbeispiel

Zum Vergleich der Bleichwirkung von zum Beispiel Natriumdithionit mit derjenigen anderer reduzierender Schwefelverbindungen wird die in Beispiel 2 beschriebene dunkelbraune Rohlösung, die einen Lichttransmissionswert von 50,4 % hat, bei 60 °C mit jeweils 0,1 Gew.-%, bezogen auf die Lösung, von Natriumdithionit (1), Natriumsulfit (2) und Natriumpyrosulfit (3) unter Rühren versetzt, worauf 1 Stunde bei einer Temperatur von etwa 60 °C nachgerührt und anschließend abgekühlt wird. Die Lichttransmissionswerte (analog Beispiel 1 bestimmt) der drei gebleichten Lösungen (1), (2) und (3) sind:
(1): 75,9 %
(2): 63,1 %
(3): 62,8 %

Die Beispiele und Vergleichsbeispiele zeigen die unerwartet hohe Bleichwirkung der erfindungsgemäß empfohlenen Schwefelverbindungen, auch wenn sie in einer nur sehr geringen Menge eingesetzt werden.

## Patentansprüche

1. Verfahren zur Herstellung von farbverbesserten Lösungen von Polyhydroxyfettsäureamiden aus N-Alkylpolyhydroxyaminen und Fettsäurealkylestern, dadurch gekennzeichnet, daß man in die Rohlösungen mindestens eine Schwefelverbindung aus der Gruppe der dithionigen Säure in einer wirksamen Menge einmischt.

2. Verfahren nach Anspruch 1, dadurch gekennzeichnet, daß man dithionige Säure oder ein Salz davon in einer wirksamen Menge einmischt.

3. Verfahren nach Anspruch 1, dadurch gekennzeichnet, daß man ein Ammoniumsalz oder Alkalimetallsalz der dithionigen Säure in einer wirksamen Menge einmischt.

4. Verfahren nach einem der Ansprüche 1 bis 3, dadurch gekennzeichnet, daß man die Schwefelverbindungen in einer Menge von 0,005 bis 1 Gew.-% einmischt, Gewichtsprozente bezogen auf den Gehalt an Polyhydroxyfettsäureamid in der Rohlösung.

5. Verfahren nach einem oder mehreren der Ansprüche 1 bis 4, dadurch gekennzeichnet, daß das Einmischen bei einer Temperatur von 20 bis 100 °C durchgeführt wird.

6. Verwendung der nach einem oder mehreren der Ansprüche 1 bis 5 erhaltenen Lösung zur Gewinnung von farbverbessertem Polyhydroxyfettsäureamid durch Isolierung des Polyhydroxyfettsäureamids aus dieser Lösung.

## Claims

1. A process for preparing a solution, of improved color, of polyhydroxy-fatty acid amide from N-alkylpolyhydroxyamine and fatty acid alkyl ester, which comprises mixing at least one sulfur compound from the group of dithionous acid into the crude solution in an active amount.

2. The process as claimed in claim 1, wherein dithionous acid or a salt thereof in an active amount is mixed in.

3. The process as claimed in claim 1, wherein an ammonium salt or alkali metal salt of dithionous acid in an active amount is mixed in.

4. The process as claimed in one of claims 1 to 3, wherein the sulfur compound is mixed in in an amount of 0.005 to 1% by weight, the percentages by weight being based on the content of polyhydroxy-fatty acid amide in the crude solution.

5. The process as claimed in one or more of claims 1 to 4, wherein the mixing in is carried out at a temperature from 20 to 100°C.

6. The use of the solution obtained as claimed in one or more of claims 1 to 5 for obtaining a polyhydroxy-fatty acid amide of improved color by isolation of the polyhydroxy-fatty acid amide from this solution.

## Revendications

1. Procédé pour la préparation de solutions à couleur améliorée d'amides d'acides gras polyhydroxyliques à partir d'alkylpolyhydroxylamines et d'esters d'alkyles d'acides gras, caractérisé en ce que l'on incorpore par mélange aux solutions brutes une quantité efficace d'au moins un composé de soufre pris dans le groupe de l'acide dithioneux.

2. Procédé selon la revendication 1, caractérisé en ce que l'on incorpore par mélange l'acide dithioneux ou un de ses sels dans une quantité efficace.

3. Procédé selon la revendication 1, caractérisé en ce que l'on incorpore par mélange un sel d'ammonium ou un sel de métal alcalin de l'acide dithioneux dans une quantité efficace.

4. Procédé selon l'une des revendications 1 à 3, caractérisé en ce que l'on incorpore par mélange les composés de soufre dans une quantité de 0,005 à 1 % en poids, les % en poids étant relatifs à la teneur en amide d'acide gras polyhydroxylique dans la solution brute.

5. Procédé selon l'une ou plusieurs des revendications 1 à 4, caractérisé en que l'on réalise l'incorporation par mélange à une température de 20 à 100 °C.

6. Utilisation de la solution obtenue selon une ou plusieurs des revendications 1 à 5, pour l'obtention d'amide d'acide gras polyhydroxylique à couleur améliorée par isolement de l'amide d'acide gras polyhydroxylique à partir de cette solution.
